# EUROPEAN PATENT SPECIFICATION

(11) **EP 0 961 135 B1**
(45) Date of publication and mention of the grant of the patent: **20.11.2002**
(21) Application number: 98105805.0
(22) Date of filing: 30.03.1998
(51) Int. Cl.: G01S 15/89

(54) **Method and apparatus for ultrasound image acquisition**
Verfahren und Vorrichtung zur Bild-Aufnahme mit Ultraschall
Procédé et appareil d'aquisition d'image par ultrasons

(43) Date of publication of application: 01.12.1999
(73) Proprietor: TomTec Imaging Systems GmbH, 85716 Unterschleissheim (DE)
(72) Inventor: Mumm, Bernhard,, 82291 Mammendorf (DE); Waldinger, Johannes,, 85579 Neubiberg (DE); Kaiser, Dietmar,, 85368 Moosburg a.d. Isar (DE); Miyamoto, Kiyoji,, Minato-ku, Tokyo 107 (JP)
(74) Representative: Müller, Hans-Jürgen, Dipl.-Ing.

(56) References cited:
- WO-A-96/13207
- US-A- 5 159 931
- US-A- 5 315 512

## Description

The present invention incorporates a method and an apparatus for obtaining three-dimensional ultrasonic, especially echographic images that allows the three-dimensional reconstruction of anatomic structures according to patent claims 1 and 11.

Three-dimensional imaging constitutes a major research goal since correct spatial structure information could help to overcome the difficult process of mental conceptualization from cross-sectional images of cardiac structures, pathology of unknown morphology and complex geometrie. Since the introduction of tomographic imaging techniques, several directions have been followed in three-dimensional ultrasound.

Presently, methods and apparatus are known for the acquisition of acquisition planes, a consecutive series of cross-sectional images using currently available standard ultrasonic transducers and retrospective "off-line" three-dimensional reconstruction. This approach necessitates the simultaneous registration of the accurate spatial position and timing of the cross-sectional images if the object under examination is in motion and if such object is to be reconstructed dynamically.

In order to acquire such acquisition planes (cross-sectional images) of internal tissues of human beings a piezoelectric transducer is used which contains an echographic probe. According to prior art (US 5 159 931) a sector-scan-transducer emits the beam of ultrasound, rotates through an angle of about 180°, with intermediate angular increments having a predetermined amplitude and frequency, around the longitudinal axis of the probe, while the latter remains fixed relative to the examined anatomic structure, there being provided means for the actuation and the control of the rotation of the scanning plane. Hence, during the rotation of the transducer a series of cross-sectional images is acquired which represent different cut planes of the object under examination. The smaller the angle steps of the rotated transducer are the more acquisition planes can be acquired and the better will be the three-dimensional reconstructed image of the internal tissue of a human being.

Positional information can be obtained with a mechanical articulated arm, an acoustic (spark gap) and magnetic location system allowing unrestricted (freehand) scanning from any available precordial acoustic window using standard transducers. Scanning techniques using a predetermined geometric acquisition pattern (linear, fan-like and rotational) allow for the recording of closely and evenly spaced cross-sectional images.

After the acquisition of such a serie of acquisition planes such cross-sectional images are digitalized and recorded together with their corresponding positions in order to process those images later on.

The recorded cross-sectional images are transformed by a transformation process into a three-dimensional data set which may be displayed later on on a display to allow doctors the diagnosis of complex diseases of the internal tissue or object.

To realize a geometrical transformation process volume-rendering algorithms are used providing grey-scale-tissue information in the reconstructions and represents a significant advance in three-dimensional echocardiography. Such echocardiographic examinations will be performed with computer-controlled transducer systems and standardized for specific conditions.

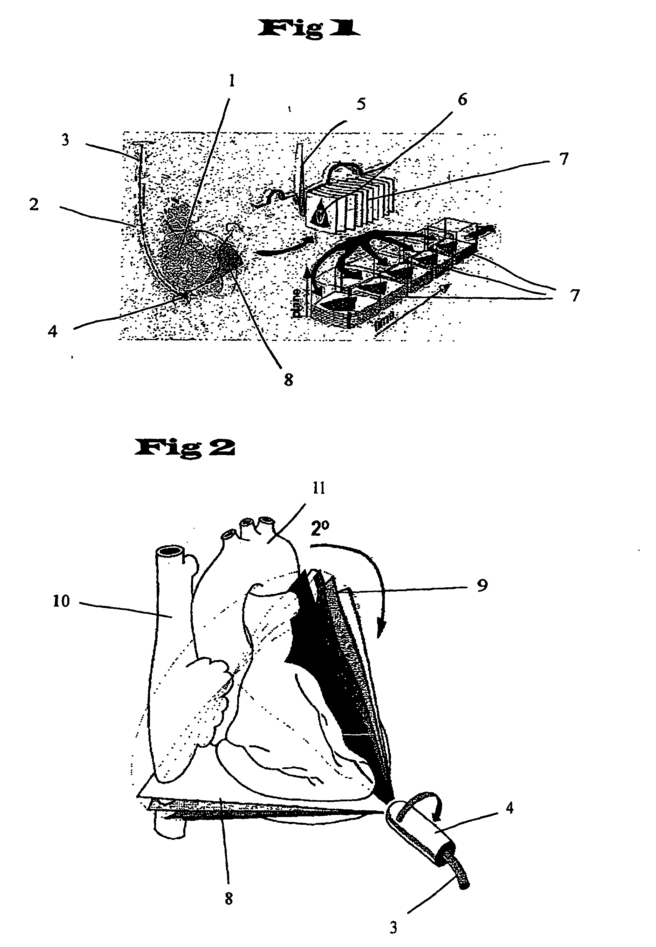

In order to investigate the shape, volume, size or motion of an organ, like the heart of a human being, prior art techniques prosecuted a complete three-dimensional acquisition by rotating the transducer in small angle steps calculating a large number of thereby acquired acquisition planes and by achieving a datacube with the whole three-dimensional image information and displaying such datacube on a suitable display. If the acquisition is a triggered image acquisition the result is a datacube with time information (compare Fig. 1).

Out of this datacube it is possible to extract cutplanes. The orientation in space and the orientation between the cut planes are known from the acquisition method. Here, by the acquisition of a fully three-dimensional reconstruction of the internal tissue it is possible to display different cutplanes through the tissue in order to investigate important regions and to detect malfunctions or diseases of the organ.

WO 96/13207 discloses said acquisition of a fully three-dimensional reconstruction of the internal tissue. The three-dimensional volume file may be subjected to various dataset reduction techniques, however, the entire three-dimensional volume file is segmented to isolate features of the organ. After such segmentation, 3-D surfaces or isosurfaces are created which may thereafter be transformed to wireframe models to enhance real-time display performance of the organ.

Roelandt, Ten Cate, Bruining, Salustri, Vletter, Mumm and Van der Putten: "Transesophageal rotoplane echo-CT - A novel approach to dynamic three-dimensional echocardiography": The Thoraxcentre Journal, Vol. 6/1, 93/94 describe a method and an apparatus of image acquisition, image processing and image display of tissue in order to investigate complex cardiac diseases. They describe the acquisition of sequential cardiac-cross sections (acquisition planes ) together with their spatial position and orientation, the image segmentation (automatic or manual boundary tracing) the realigning and combining of the cross-sections into a three-dimensional structure at either the selected points or throughout the cardiac cycle (dynamically), the interpolation of data "missing" between individual cross-sections and the displaying of three-dimensional images (see also US 5 159 931). Here, it is described to use a step-motor via a custom-built wheel-work interface to rotate the control knob of the multiplane probe. The step motor is activated by the steering logic which controls the image acquisition in a given plane by an algorithm considering heart cycle variation by ECG-gating and respiratory cycle variation by impedance measurement. This allows optimal temporal and spatial registration of the cardiac cross-sections. Here, the probe is moved by small angular increments for a predetermined number of times (e.g. 2°, 90 cross-sections per tissue) in order to acquire a full three-dimensional reconstruction.

However, difficulties in obtaining high quality cardiac tomographic images in multiple orientations by precordial echocardiography and the time-consuming three-dimensional reconstruction procedure requiring observer tracing of the boundaries of interest are major limitations and have hampered the clinical implementation of three-dimensional echoca rdiography up to now.

Additionally, by introducing an endoscope into the vene of a patient implies pain so that the acquisition time should be as short as possible. If the patient is moving during the acquisition or if the respiration is uneven due to the pain arising throughout the endoscope dynamic artefacts diminish the quality of the three-dimensional reconstructed image of the internal organ of the patient.

Even more, it is sometimes sufficient for the evaluation of a possible organ disease to obtain information about the size, the shape, the volume and the movement of internal organs.

The object of the invention is to obtain a three-dimensional reconstruction of objects in a more rapid and effective way and to achieve suitable information of the condition of an organ without using the time consuming reconstruction procedures of prior art and without the major limitations of three-dimensional echocardiography incorporated within prior art techniques.

It is another object of the invention to rapidly acquire dynamic and quantitative information of an internal organ by using low cost apparatus and at the same time facilitating the acquisition methods for non-professionals.

The objects are solved throughout the characterizing features of claims 1 and 11. Preferable embodiments are claimed within the sub-claims and are further specified as follows:

The claimed method for obtaining three-dimensional echographic images comprises the steps of scanning an object by two-dimensional image scanning means in order to acquire acquisition planes of the object Those image scanning means are moved in predetermined increments for a predetermined number of times or homogeneously along said object (freehand acquisition) while scanning said object and digitalizing each of said cross-sectional acquisition planes. Those digitalized cross-sectional images are recorded together with their corresponding positions. Those recorded cross-sectional images are transformed into a three-dimensional data set by a transformation process and may thereafter be displayed on a suitable display.

Thereafter, the contours of the object within cross-sectional images are detected, wherein said cross-sectional images are said acquisition planes and/or cutplanes of said three-dimensional data set. Afterwards a wireframe volume model is created which represents the three-dimensional data set by using the positions of respective cross-sectional images and the contours within said cross-sectional images. For the acquisition of a wireframe volume model much less cross-sectional images are necessary than for the acquisition of a full three-dimensional reconstructed image of the tissue.

Such wireframe volume model is created by linking a contour point of said contours, which represent three-dimensional coordinates, by using surface acquisition or volume rendering techniques. This contour detection is performed by a semi-automatic, automatic or manual contour detection algorithm.

The result will be a three-dimensional wireframe which can be displayed in a surface rendering mode or for example as a semi-transparent overlay in a three-dimensional datacube which has been acquired in before. The respective contours of the object under examination can be detected by using grey level ranges followed by the application of noice reduction algorithms, edge enhancement algorithms and/or spatial artifacts reduction algorithms. After such contours have been detected the respective contour points of special interest can be linked with horizontal and vertical wire-lines, preferably with interpolation algorithms, in order to finally represent the wireframe volume model. Alternatively, it is possible to link such contour points, with all adjacent contour points thereby achieving a wireframe model which consists of even triangles representing the three-dimensional surface of the tissue.

For the acquisition of echocardiographic images of cardiac structures, especially for the acquisition of an image representing the heart of a human being, it is possible to create several wireframe volume models of the same object in motion. Each wireframe volume model represents one condition of the cardiac tissue. By adding the dimension of time for functional assessment these different wireframe volume models can be displayed successively to acquire dynamic or quantitative information of the object, thus representing a "moving" wireframe model. Hence, it is possible to measure e.g. quantitatively the left ventricular function of the heart, the ejaction fraction or the wall motion. Diseases or malfunctions of the heart can thus be easily detected.

In order to properly display the successive wireframe models it is necessary to display the three-dimensional data sets in accordance with an algorithm considering heart cycle variations by ECG-gating and respiratory cycle variations by impedance measurements. A rotoplane echo-CT wireframe acquisition method comprises the moving of the image scanning means in predetermined angular increments by only acquiring the most important cut planes of the tissue under examination (e.g. for a left ventricle rotation acquisition ever 30° rot angle) for a predetermined number of times to record each of such cross-sectional images and its corresponding angular position and to detect the contours of the object within said cross-sectional images with respect to a common rotation axis of said cross-sectional images .

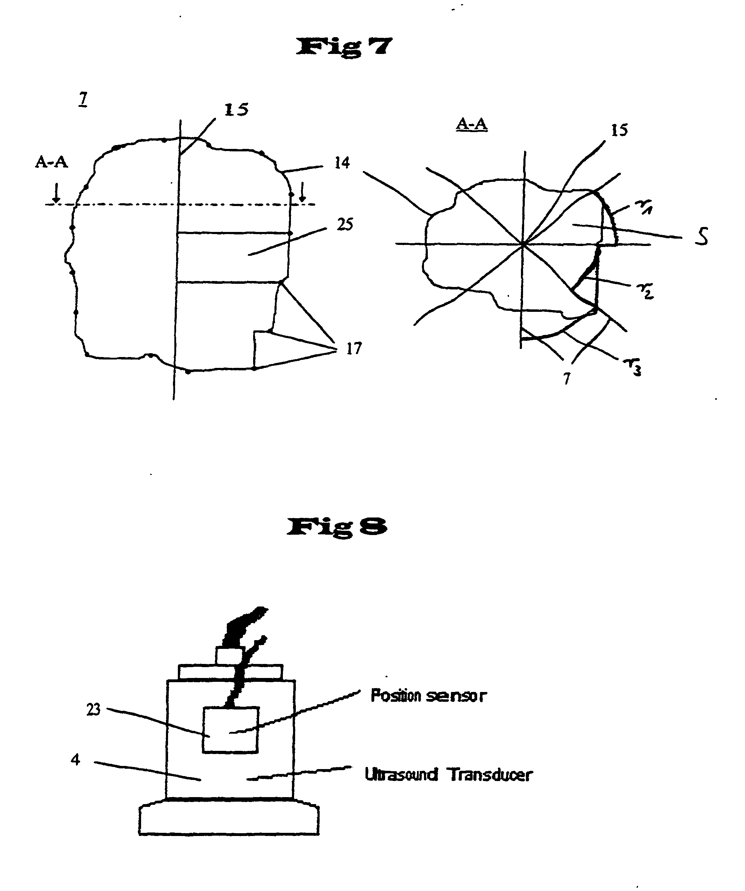

Additionally, it might be useful to record one or more cross-sectional images from cutplanes of the object which are substantially orthogonal to said angular positioned cross-sectional images by relocating the transducer to another place or by using another e.g. handheld-transducer (Fig. 7). Consequently, it is easier to link the respective contour points by specifying special contour points represented by the cross-sections of a rotoplane (angular cross-section) and an orthogonal cutplane (relocated acquired cross-section).

The corresponding apparatus for obtaining three-dimensional echographic images comprises two-dimensional image scanning means for scanning an object in order to acquire acquisition planes of said object and means for moving that image scanning means in predetermined increments for a predetermined number of times or homogeneously along said object. Digitalization means digital ize the acquired cross-sectional images and recording means record these digitalized cross-sectional images and their corresponding positions. Afterwards transforming means transform these cross-sectional images into a three-dimensional data-set by a geometrical transformation process wherein such three-dimensional data set is then displayed by displaying means.

Then, detecting means detect the contours of said object within cross-sectional images, wherein said cross-sectional images are said acquisition planes and/or cutplanes of said three-dimensional data set, by implementing an incremental or dynamic threshold to differentiate lighter or darker sections of the grey leveled ultrasonic image. Volumetric acquisition means thereafter create a wireframe volume model by using the positions of the respective cross-sectional images and the detected contours or contour points which represent three-dimensional coordinates within said cross-sectional images.

If the image scanning means are incrementally moved the apparatus further incorporates a step-motor and a steering logic to control the incremental acquisition of acquisition planes of the object. Furthermore, the two-dimensional scanning means comprise a position sensor for detecting the position of the respective cross-sectional acquisition planes which is important for a freehand acquisition by moving the transducer homogeneously along the object under examination.

For the dynamic acquisition the apparatus further comprises ECG-gating means and respiration trigger means for dynamically scanning cardiac objects in order to be able to display the moving tissue in motion in cine-loop format.

A preferred embodiment of the present invention and a routine cardiac ultrasound examination, rapid three-dimensional acquisition method will be explained in conjunction with the drawings as follows:
- Fig. 1: shows an endoscope echocardiographical acquisition,
- Fig. 2: shows the principle of acquisition of sequential acguisition planes of a heart,
- Fig. 3: shows rotated cutplanes,
- Fig. 4: shows a cross-sectional image with detected contours,
- Fig. 5: shows rotated cutplanes with the contour information,
- Fig. 6: shows a calculated wireframe volume model,
- Fig. 7: shows the average rotation volume acquisition technique,
- Fig. 8: shows a freehand ultrasound transducer,
- Fig. 9: shows a motor-controlled rotation transducer, and
- Fig. 10: shows the transducer of Fig. 8 with a position sensor.

Fig. 1 shows an object (1) like an internal organ or tissue (e.g. heart) which is scanned by an ultrasonic beam (8) from scanning means (4) like a transducer which is incorporated within a probe situated in a vene or artery adjacent to the object (1 wherein the scanning means (4) are linked with processing means (not shown) by an endoscope connection (3) within the endoscopical path (2) (e.g. vene or artery). The transesophageal probe (4) houses e.g. a 5 MHz 64-element rotational-array transducer at the distal end of a standard gastroscope or endoscope. The dimensions of the multiplane probe (4 ) are e.g. 14 mm width, 10,3 mm thickness and 40 mm length. The scanning plane can be continuously rotated through 180° starting from a longitudinal imaging position via a control knob on the handle of the echoscope. The cardiac cross-sections encompass a cone shaped volume with its point originating in the transducer (compare also Fig. 2). After positioning in the esophagus the probe (4) can be kept in a steady position by locking the antero-posterior flexion control in the anterior position.

Then a step motor is activated and mechanically rotates the probe (4) which allows optimal temporal and spatial registration of the cardiac cross-sections.

The step motor is activated by the steering logic which controls the image acquisition in a given plane by an algorithm considering heart cycle variation by ECG-gating (5) and respiratory cycly variation by impedence measurement. For each position of the heart (e.g. systole oder diastole) the heart is scanned by acquiring a series of cross-sections (6), thereby obtaining a set of acquisition planes (9) which belong to a certain position of the heart (stroposcopical picture).

When a cardiac cycle is selected by the steering logic the cardiac cross sections (6) are sampled at e.g. 40 msec intervals during a complete heart cycle, digitized and stored in the computer memory. Then, the step motor is activated and rotates the control knob 30° to the next cross-section (6) where the same steering logic is followed. To encompass the whole heart (or region) six sequential standardized cross-sections from 0-180° must be obtained each during a complete heart cycle.

Fig. 2 shows an enlarged few of the principle of acquisition of sequential cross-sections (6) from the apical transducer position. A transducer (4) being linked to the computer by an endoscope connection (3) scanns the heart (1) consisting of the right ventricle (10) and the left ventricle (11) by acquiring a couple of acquisition planes (9) troughout the ultrasonic beam (8).

During the acquisition phase the cardiac cross sections are digitized and stored in the computer memory. In the post-processing phase they are formatted in the correct sequence (compare Fig. 1) according to their ECG-phase in volumetric data sets.

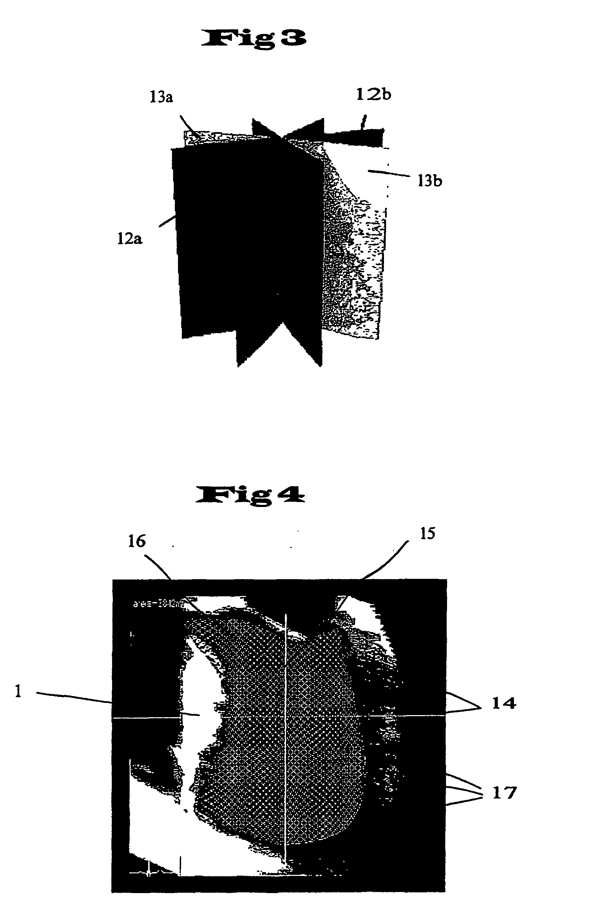

Fig. 3 shows four rotated cutplanes (12a, 12b, 13a, 13b) being rotational positioned around a common rotation axis (15).

Fig. 4 shows a cross-section (6) with a contour (14) which is based on contour points (17) thus defining a contour surface (16). These contour points (17) are connected with a spline-interpolation algorithm. If the object is not in the rotation axis (15) it is possible to define a new rotation axis (15).

A grey level range is used to separate cardiac structures from the bloodpool and background in each cross-section (6) followed by the application of several algorithms to reduce noise, to enhance edges and the reduction of spatial artifacts (ROSA filter) . Thus, the contour (14) can be defined.

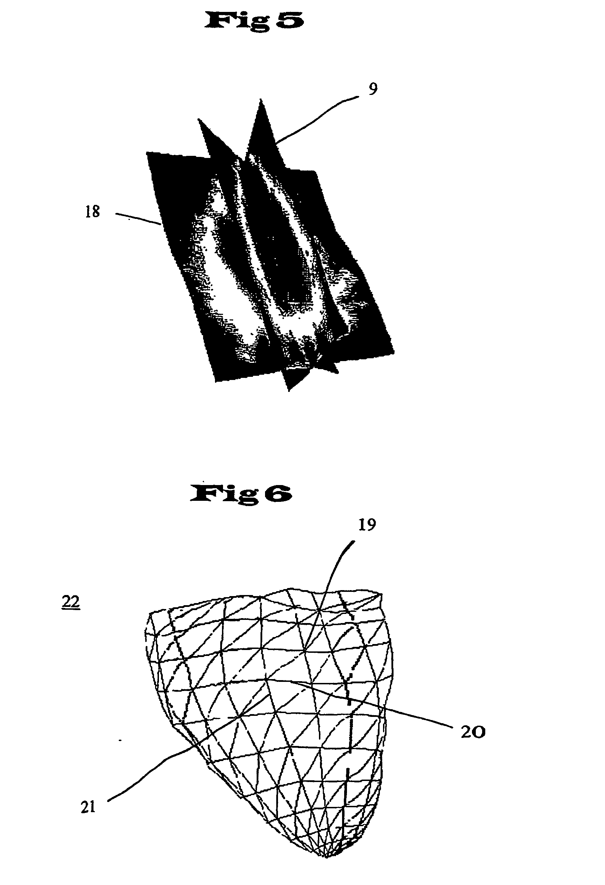

Fig. 5 shows rotated acquisition planes (9) representing the above specified cutplanes (12a, 12b, 13a, 13b) being positioned around a common rotation axis (15) together with the contour information (14) being detected in before. A 3D-data cube (18) is hence virtually situated among the acquisition planes (9) .

Fig. 6 shows the calculated wireframe volume model (22) with the contour points (19), horizontal wirelines (20) and vertical wirelines (21). The calculated contour points (17, 19) reperesent 3D-coordinates within such wire-frame volume model (22) and have to be linked by surface acquisition or volume rendering techniques. Here, it is possible to link each contour point (19) with all adjacent contour points thus obtaining a large number of small triangles representing the surface of the wireframe volume model (22).

The acquisition method of the present invention requires only a couple of cutplanes (12a, 12b, 13a, 13b) a fast and effective detection of contour points (17, 19) and the calculation of the wireframe volume model (22) in order to obtain a good approximation of the object (1) in order to get dynamic, quantitative or shape information.

Fig. 7 shows a technique for acquiring the volume of the object (1). The rotation axis (15) of a cross sectional image (7) is defined through the centre of the object (1). About the rotation axis (15) N acquisition planes (9) or cutplanes (12a, 12b, 13a, 13b), as cross-sectional images (7), are defined with respect to the previous (Fig. 2) acquisition. Each plane represents a rotation of 180/N degrees with respect to the previous cutplane. A contour (14) with contour points (17) is defined for each cross-sectional image (7). The rotation axis (15) cuts each cross-sectional image (7) in two. In order to acquire the volume of the object (1), the method calculates the rotation volumes V(1...2N) for both parts of each cross-sectional image (7). The rotation volume V is defined by a segment (S), consisting of the rotation axis (15) , the contour (14) of one half of a cross-sectional image (7) being rotated about 180/N degrees (r₁, r₂, r₃, ... r_{N}) until the contours (14) of the next cross-sectional image (7), the next cutplane (9), is reached. To be more precise it is also possible to rotate only segments (25) of one part of a cross sectional image (7), those segments (25) being defined by 2 successively situated contour points (17) of said contour (14) and the rotation axis (15). To get the volume of the whole object (1), all rotation volumes of said segments (25) are added.

For symmetrical rotational objects, only 1 cutplane (12, 13) would be enough to get the exact volume. The exact measurement of the contours. (14) depends on the number of contour points (17) which can be increased by using spline curves. The rotation axis (15) should be defined by the largest dimension of the object (1) in order to get good and fast results. The more simple the object (1) is shaped, the smaller the number of cutplanes (12, 13) may be in order to achieve good and fast results. The exact approximation of the volume depends on the number of cuts.

The same acquisition method is also possible for successively acquired acquisition planes (9) linearily along the object (1) by free-hand scanning or machine-controlled scanners. The volume is acquired by moving said contours (14) or segments (25) of said contours (14) within said cross-sectional images (7) along an axis which is substantially perpendicular to said cross-sectional images until the contours (14) of the next cross-sectional image (7), the next acquisition planes (9), is reached. Here, the movement is not rotational but linear since the acquisition planes (9) were successively acquired linearily along the object (1).

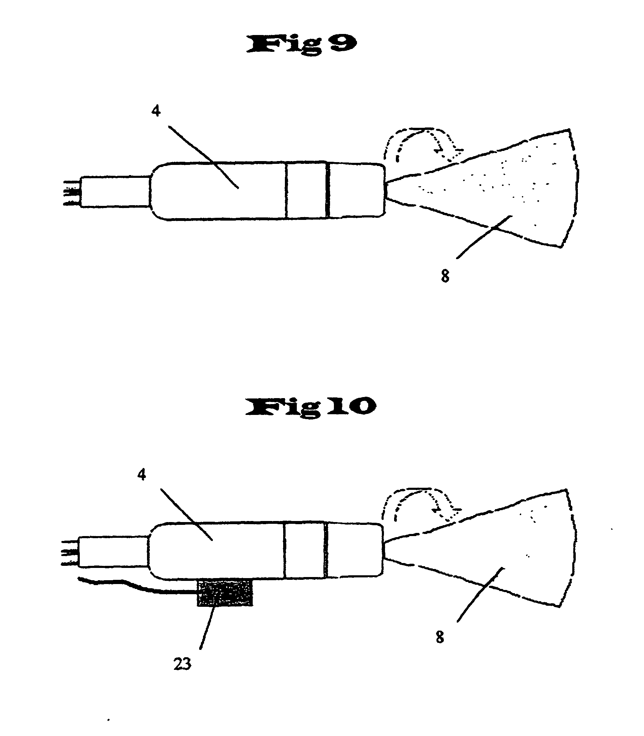

Fig. 8 shows a freehand transducer (4) together with a position sensor (23).

Fig. 9 shows a rotational transducer (4) as shown in Fig. 2 admitting an ultrasonic beam (8) which is rotating in predetermined incremental steps or homogeneously, thereby scanning the object (1) under examination.

Fig. 10 shows the transducer (4) of Fig. 8 together with a position sensor (23). The position sensor (23) is useful for detecting the rotation axis (15) if the transducer (4) is slightly moved during the acquisition of the acquisition planes (9). The information of the position sensor (23) can be computed in order to realigne and to correctly locate the acquisition planes (9) with respect to each other.

For the rapid three-dimensional acquisition method of the present invention the following four acquisition methods are possible :
1. A freehand acquisition with the transducer (4) of Fig. 7 and the position sensor (23) is possible by acquiring different acquisition planes (9) wherein the position sensor (23) supplies the transformation means with the spatial orientation of the different acquisition planes (9).
2. Furthermore, a triggered freehand acquisition is possible wherein a transducer (4) together with a position sensor (23) is used which is the same method as described above but with an ECG- and respiration triggered acquisition.
3. A motor controlled rapid acquisition with a rotational transducer (4) as shown in Fig. 8 and 9 is feasable as well as
4. a motor controlled rapid acquisition together with a position sensor (23). The position sensor (23) enables the acquisition apparatus to locate the proper rotation axis (15) so that movements of the rotational ultrasound probe (4) do have no influence on the image calculation.

The rapid three-dimensional aquisition method of the present invention is also feasable for color Doppler examination as follows:

In the routine cardiac ultrasound examination the doctor evaluates the disease of Mitral Valve Regurgitation (MR), Aortic Valve Regurgitation (AR), Mitral Valve Stenosis (MS) Aortic Valve Stenosis (AS) etc. using color Doppler examination. These patients have a very fast and turbulent blood flow (Jet flow). Normally Jet flow is represented by a mosaic pattern in the color Doppler image. The doctor examining the patient tries to visualize the biggest Jet flow by changing the probe position.

In the routine the echo doctor evaluates the severity of disease by measuring the area (e.g. regurgitant area) of Jet flow (mosaic pattern). This evaluation is done by only one cut plane. By using a three-dimensional color flow acquisition using a freehand scan method it is possible to evaluate the valvular disease more accurately that means closer to the actual flow information even if errors occure.

The procedure is done as follows:
1. The biggest Jet flow is visualized by using the color Doppler mode.
2. At the same transducer probe position one cardiac cycle is acquired by using a freehand scan method together with ECG-gating.
3. The transducer probe is rotated to a certain degree and another cardiac cycle is acquired. These acquisitions are continued several times in order to obtain 2-20, preferably 3-6, cardiac cycle slices.
4. During the acquisition the rotation axis and the position of the probe will be inclined wherein all angular and positional information will be recorded together with the colour Doppler images.
5. The rotation axis is compensated or realigned for each image ( each phase) to the first position of the biggest Jet flow.
6. The best phase for the evaluation is selected.
7. A manual, semi-automatic or automatic detection of the Jet flow area (mosaic pattern) or the border of each image is conducted.
8. The three-dimensional color flow volume is calculated and the severity of the valvular disease is evaluated.
9. Another possibility is to surface render one cardiac cycle color flow image from a certain view by using a wireframe volume model of the present invention.

## Claims

1. Method for obtaining three-dimensional ultrasound images, comprising the steps of
- scanning an object (1) by two-dimensional image scanning means (4) to acquire acquisition planes (9) of said object (1),
- moving said image scanning means (4) in predetermined increments for a predetermined number of times or homogeneously along said object (1), while scanning said ooject (1),
- digitalizing each of said acquisition planes (9),
- recording each of said acquisition planes (9) and its corresponding position, and
- transforming said acquisition planes (9) into a three-dimensional data set by a transformation process, and
- displaying said three-dimensional data set,
**characterized by**
realizing said transformation process by
- detecting the contours (14) of said object (1) within cross-sectional images (7), wherein said cross-sectional images (7) are said acquisition planes (9) and/or cutplanes (12, 13) of said three-dimensional data set,
- creating a wireframe volume model (22) by using the positions of respective cross-sectional images (7) and said contours (14) within said cross-sectional images (7)

2. Method as claimed in claim 1,
**characterized by**
linking contour points (17, 19) of said contours (14) by using surface acquisition and/or volume rendering techniques.

3. Method as claimed in claim 2,
**characterized by**
linking contour points (17, 19) of said contours (14) with horizontal and vertical wire lines (20, 21), preferably with an interpolation algorithm.

4. Method as claimed in claim 2,
**characterized by**
linking contour points (17, 19) of said contours (14) by linking each of said contour points (19) with all adjacent contour points (19).

5. Method as claimed in claim 2,
**characterized by**
moving or rotating said contours (14) or segments (25) of said contours (14) within said cross-sectional images (7) along an axis, being substantially perpendicular to said cross-sectional images (7), or about a common rotation axis (15) of said cross-sectional images (7) until the contours (14) of the next cross-sectional image (7) are reached.

6. Method as claimed in one of the preceding claims,
**characterized by**
detecting the contours (14) of said object (1) by using grey level ranges, followed by the application of noise reduction algorithms, edge enhancement algorithms and/or spatial artifacts reduction algorithms .

7. Method as claimed in one of the preceding claims,
**characterized by**
creating several wireframe volume models (22) of an object (1) in motion and displaying said three-dimensional data sets, which correspond to said wireframe volume models (22), successively, to acquire dynamic and/or quantitative information of said object (1).

8. Method as claimed in one of the claims 1 to 6,
**characterized by**
creating several wireframe volume models (22) of a cardiac object (1) in motion and displaying said three-dimensional data sets in accordance with an algorithm considering heart cycle variations by electrocardiographic gating and respiratory cycle variations by impedance measurements.

9. Method as claimed in one of the preceding claims,
**characterized by**
moving said image scanning means (4) in predetermined angular increments for a predetermined number of times,
recording each of said acquisition planes (9) and its corresponding angular position, and
detecting the contours (14) of said object (1) within cross-sectional images (7) with respect to a common rotation axis (15) of said cross-sectional images (7).

10. Method as claimed in claim 9,
**characterized by**
additionally recording one or more cross-sectional images (7) from cutplanes (12,13) of said object (1) which are substantially orthogonal to said angular positioned cross-sectional images (7).

11. Apparatus for obtaining three-dimensional echographic images, comprising
- two-dimensional image scanning means (4) for scanning an object (1) to acquire acquisition planes (9) of said object (1),
- moving means for moving said image scanning means (4) in predetermined increments for a predetermined number of times or homogeneously along said object (1),
- digitalization means for digitalize each of said acquisition planes (9),
- recording means for recording each of said acquisition planes (9) and its corresponding position,
- transforming means for transforming said acquisition planes (9) into a three-dimensional data set by a geometrical transformation process, and
- displaying means for displaying said three-dimensional data set,
**characterized by,**
said transforming means comprising
- detecting means for detecting the contours (14) of said object (1) within cross-sectional images (7), wherein said cross-sectional images (7) are said acquisition planes (9) and/or cutplanes (12, 13) of said three-dimensional data set, and
- volumetric acquisition means for creating a wireframe volume model (22) using said positions of said cross-sectional images (7) and said contours (14) within said cross-sectional images (7).

12. Apparatus as claimed in claim 11,
**characterized in that**
said means for moving said image scanning means (4) incooperate a step motor and a steering logic which control the incremental acquisition of acquisition planes (9) of said object (1).

13. Apparatus as claimed in claim 11 or 12,
**characterized in that**
said two-dimensional image scanning means (4) comprise a position sensor (23) for detecting the positions of said acquisition planes (9).

14. Apparatus as claimed in one of the claims 11 - 13,
**characterized in that**
said apparatus further comprises electrocardiographic gating means and respiration trigger means for dynamically scanning cardiac objects (1).

## Patentansprüche

1. Verfahren zur Aufnahme von dreidimensionalen Ultraschallbildern, welches die folgenden Schritte aufweist:
- Abtasten eines Gegenstands (1) mittels einer Einrichtung (4) zur zweidimensionalen Abtastung, um Aufnahmeebenen (9) für das Objekt (1) zu erhalten,
- Bewegen der Bildabtasteinrichtung (4) in vorgegebenen Schritten eine vorgegebene Anzahl von Malen oder homogen entlang des Objekts (1) unter Abtastung des Objekts (1),
- Digitalisieren jeder der Aufnahmeebenen (9),
- Aufzeichnen jeder der Aufnahmeebenen (9) und deren entsprechender Position, und
- Umwandeln der Aufnahmeebenen (9) in einen dreidimensionalen Datensatz mittels eines Umwandlungsprozesses, und
- Anzeigen des dreidimensionalen Datensatzes,
**gekennzeichnet durch:**
- Ausführen des Umwandlungsprozesses **durch** Erfassen der Umrisslinien des Objekts (1) innerhalb von Querschnittsbildern (7), wobei die Querschnittsbilder (7) Aufnahmeebenen (9) und/oder Schnittebenen (12, 13) des dreidimensionalen Datensatzes sind,
- Erstellen eines Drahtmodells (22) als Volumenmodell unter Verwendung der Positionen jeweiliger Querschnittsbilder (7) und der Umrisslinien (14) innerhalb der Querschnittsbilder (7).

2. Verfahren nach Anspruch 1,
**gekennzeichnet durch**
die Verknüpfung von Umrisspunkten (17, 19) der Umrisslinien (14) unter Heranziehung von Techniken zur Aufnahme von Oberflächen und/oder zur Wiedergabe eines Volumens.

3. Verfahren nach Anspruch 2,
**gekennzeichnet durch**
die Verknüpfung von Umrisspunkten (17, 19) der Umrisslinien (14) mit horizontalen und vertikalen Drahtmodell-Linien (20, 21), vorzugsweise mit einem Interpolations-Algorithmus.

4. Verfahren nach Anspruch 2,
**gekennzeichnet durch**
die Verknüpfung von Umrisspunkten (17, 19) der Umrisslinien (14) **durch** Verknüpfen jedes der Umrisspunkte (19) mit allen benachbarten Umrisspunkten (19).

5. Verfahren nach Anspruch 2,
**gekennzeichnet durch**
Bewegen oder Drehen der Umrisslinien (14) bzw. von Segmenten (25) der Umrisslinien (14) innerhalb der Querschnittsbilder (7) entlang einer Achse, welche im wesentlichen senkrecht zu den Querschnittsbildem (7) verläuft, oder um eine gemeinsame Drehachse (15) der Querschnittsbilder (7), bis die Umrisslinien (14) des nächsten Querschnittsbildes (7) erreicht sind.

6. Verfahren nach einem der vorhergehenden Ansprüche,
**gekennzeichnet durch**
die Erfassung der Umrisslinien (14) des Objekts (1) unter Verwendung von Graustufenbereichen, mit anschließender Anwendung von Algorithmen zur Verringerung des Rauschens, von Algorithmen zur Verbesserung der Ränder, und/oder Algorithmen zur Verringerung von räumlichen Artefakten.

7. Verfahren nach einem der vorhergehenden Ansprüche,
**gekennzeichnet durch**
die Erstellung mehrerer Drahtmodelle als Volumenmodelle (22) eines Objekts (1) in dessen Bewegung und Anzeigen der dreidimensionalen Datensätze nacheinander, welche den Drahtmodellen (22) entsprechen, um so dynamische und/oder quantitative Informationen über das Objekt (1) zu erhalten.

8. Verfahren nach einem der Ansprüche 1 bis 6,
**gekennzeichnet durch**
die Erstellung mehrerer Drahtmodelle als Volumenmodelle (22) eines Herzobjekts (1) in dessen Bewegung und Anzeigen der dreidimensionalen Datensätze entsprechend einem Algorithmus, bei welchem Schwankungen im Herzschlagzyklus **durch** elektrokardiographische Ausblendung und Schwankungen im Atmungszyklus **durch** Impedanzmessungen berücksichtigt werden.

9. Verfahren nach einem der vorhergehenden Ansprüche,
**gekennzeichnet durch**
das Bewegen der Bildabtasteinrichtung (4) um vorgegebene Winkelschritte über eine vorgegebene Anzahl von Malen,
das Aufzeichnen jeder der Aufnahmeebenen (9) und deren entsprechender Winkelposition, und
das Erfassen der Umrisslinien (14) des Objekts innerhalb von Querschnittsbildem (7) bezüglich einer gemeinsamen Drehachse (15) der Querschnittsbilder (7).

10. Verfahren nach Anspruch 9,
**gekennzeichnet durch**
das zusätzliche Aufzeichnen eines Querschnittsbildes oder mehrerer Querschnittsbilder aus Schnittebenen (12, 13) des Objekts (1), die im Wesentlichen senkrecht zu den unter einem Winkel positionierten Querschnittsbildern (7) verlaufen.

11. Vorrichtung zum Aufzeichnen von dreidimensionalen echographischen Bildern, welche folgendes aufweist:
- eine Bildabtasteinrichtung (4) zum Abtasten zweidimensionaler Abbildungen, welche das Objekt (1) abtastet, um so Aufnahmeebenen (9) des Objekts (1) aufzunehmen,
- eine Bewegungseinrichtung zum Bewegen der Bildabtasteinrichtung (4) in vorgegebenen Schritten über eine vorgegebene Anzahl von Malen oder homogen entlang des Objekts (1),
- eine Digitalisierungseinrichtung zum Digitalisieren jeder der Aufnahmeebenen (9),
- eine Aufzeichnungseinrichtung zum Aufzeichnen jeder der Aufnahmeebenen (9) und deren entsprechender Position,
- eine Umwandlungseinrichtung zum Umwandeln der Aufnahmeebenen (9) in einen dreidimensionalen Datensatz mittels eines Prozesses zur geometrischen Umwandlung, und
- eine Anzeigeeinrichtung zum Anzeigen des dreidimensionalen Datensatzes,
**dadurch gekennzeichnet, dass**
- die Umwandlungseinrichtung eine Erfassungseinrichtung zum Erfassen der Umrisslinien (1 4) des Objekts (1) innerhalb von Querschnittsbildern (7) aufweist, wobei die Querschnittsbilder (7) die Aufnahmeebenen (9) und/oder Schnittebenen (12, 13) des dreidimensionalen Datensatzes sind, und
- dass eine Einrichtung zur volumetrischen Aufnahme zum Erstellen eines Drahtmodells (22) als Volumenmodell unter Verwendung der Positionen der Querschnittsbilder (7) und der Umrisslinien (14) innerhalb der Querschnittsbilder (7) vorgesehen ist.

12. Vorrichtung nach Anspruch 11,
**dadurch gekennzeichnet, dass**
die Einrichtung zum Bewegen der Bildabtasteinrichtung (4) einen Schnittmotor und eine Steuerlogik umfasst, welche die schrittweise Erfassung von Aufnahmeebenen (9) des Objekts (1) steuern.

13. Vorrichtung nach Anspruch 11 oder 12,
**dadurch gekennzeichnet, dass**
die Bildabtasteinrichtung (4) einen Positionsfühler (23) zum Erfassen der Positionen der Aufnahmeebenen (9) aufweist.

14. Vorrichtung nach einem der Ansprüche 11 bis 13,
**dadurch gekennzeichnet, dass**
die Vorrichtung des Weiteren eine Einrichtung zur elektrokardiographischen Ausblendung und eine Auslöseeinrichtung zum Auslösen der Atmung für eine dynamische Abtastung von Herzobjekten (7) aufweist.

## Revendications

1. Procédé d'acquisition d'images ultrasonores, qui comprend les opérations suivantes :
- l'exploration d'un objet (1) moyennant un moyen (4) d'exploration bidimensionnelle d'images afin d'obtenir des plans d'acquisition (9) pour l'objet (1),
- le mouvement dudit moyen (4) d'exploration d'images par des incréments déterminés en un nombre prédéterminé de fois ou de façon homogène le long de l'objet (1), en explorant ledit objet (1),
- numérisation de chacun desdits plans d'acquisition (9),
- enregistrement de chacun desdits plans d'acquisition (9) et de sa position correspondante, et
- transformation desdits plans d'acquisition (9) en un en un ensemble de données tridimensionnelles moyennant un processus de transformation, et
- affichage de l'ensemble de données tridimensionnelles,
**caractérisé par**:
- la réalisation du processus de transformation par acquisition des lignes de contour de l'objet (1) au-dedans des images en coupe transversale (7), lesdites images en coupe transversale (7) étant des plans d'acquisition (9) et/ou des plans de coupe (12, 13) de l'ensemble de données tridimensionnelles,
- établissement d'un modèle à grille en fils (22) en tant que modèle de volume, en utilisant les positions des images respectives en coupe transversale (7) et desdites lignes de contour (14) au-dedans desdites images (7) en coupe transversale.

2. Procédé selon la revendication 1,
**caractérisé par**
le chaînage des points de contour (17, 19) desdites lignes de contour (14), en utilisant des techniques d'acquisition de surfaces et/ou de reproduction d'un volume.

3. Procédé selon la revendication 2,
**caractérisé par**
le chaînage des points de contour (17, 19) desdites lignes de contour (14) par des lignes horizontales et verticales (20, 21) dudit modèle à grille en fils, de préférence par un algorithme d'interpolation.

4. Procédé selon la revendication 2,
**caractérisé par**
le chaînage des points de contour (17, 19) desdites lignes de contour (14) en chaînant chacun desdits points de contour (19) avec tous les points de contour adjacents (19).

5. Procédé selon la revendication 2,
**caractérisé par**
le mouvement ou la rotation desdites lignes de contour (14) ou respectivement des segments (25) desdites lignes de contour (14) au-dedans desdites images en coupe transversale (7) le long d'un axe, qui s'étend essentiellement en sens orthogonal sur lesdites images en coupe transversale (7) ou autour d'un axe commun de rotation (15) desdites images en coupe transversale (7), jusqu'à ce qu'on arrive auxdites lignes de contour (14) de l'image en coupe transversale suivante (7).

6. Procédé selon une quelconque des revendications précédentes,
**caractérisé par**
l'acquisition desdites lignes de contour (14) de l'objet (1), en utilisant des gammes d'échelles des gris, à application suivante des algorithmes à réduire le bruit, des algorithmes à améliorer les bords, et/ou des algorithmes à réduire des artéfacts spatiaux.

7. Procédé selon une quelconque des revendications précédentes,
**caractérisé par**
l'établissement de plusieurs modèles à grille en fils en tant que modèles de volume (22) d'un objet (1) au cours du mouvement du dernier, et l'affichage des ensembles de données tridimensionnelles en succession, qui correspondent aux modèles à grille en fils (22), afin d'obtenir ainsi des informations dynamiques et/ou quantitatives sur l'objet (1).

8. Procédé selon une quelconque des revendications 1 à 6,
**caractérisé par**
l'établissement de plusieurs modèles à grille en fils en tant que modèles de volume (22) d'un objet cardiaque (1) au cours du mouvement du dernier, et l'affichage des données tridimensionnelles en correspondance avec un algorithme, dans lequel on prend en considération des variations du cycle cardiaque par extraction électrocardiographique et les variations dans le cycle respiratoire par mesures d'impédance.

9. Procédé selon une quelconque des revendications précédentes,
**caractérisé par**
le mouvement dudit moyen d'exploration d'images (4) par des pas angulaires prédéterminés en un nombre prédéterminé de fois,
l'enregistrement de chacun desdits plans d'acquisition (9) et leur position angulaire respective, et
l'acquisition desdites lignes de contour (14) de l'objet au-dedans des images en coupe transversale (7) relativement à un axe commun de rotation (15) desdites images en coupe transversale (7).

10. Procédé selon la revendication 9,
**caractérisé par**
l'enregistrement supplémentaire d'une ou plusieurs images en coupe transversale à partir des plans de coupe (12, 13) de l'objet, qui s'étend essentiellement en sens orthogonal sur lesdites images en coupe transversale (7), qui sont positionnées à un angle.

11. Appareil d'acquisition d'images tridimensionnelles échographiques, qui comprend:
- un moyen d'exploration d'images (4) à explorer des images bidimensionnelles, qui analyse l'objet (1) afin d'acquérir des plans d'acquisition (9) de l'objet (1),
- un moyen de mouvement pour le mouvement dudit moyen d'exploration d'images (4) par des incréments déterminés en un nombre prédéterminé de fois ou de façon homogène le long de l'objet (1),
- un moyen de numérisation à numériser chacun desdits plans d'acquisition (9),
- un moyen enregistreur à enregistrer chacun desdits plans d'acquisition (9) et sa position correspondante,
- un moyen de transformation à transformer lesdits plans d'acquisition (9) en un ensemble de données tridimensionnelles par un processus de transformation géométrique, et
- un moyen d'affichage à afficher l'ensemble de données tridimensionnelles,
**caractérisé en ce**
- **que** ledit moyen de transformation comprend un moyen d'acquisition à acquérir lesdites lignes de contour (1 4) de l'objet (1) au-dedans des images en coupe transversale (7), lesdites images en coupe transversale (7) étant lesdits plans d'acquisition (9) et/ou des plans de coupe (12, 13) de l'ensemble de données tridimensionnelles, et
- en ce qu'un moyen d'acquisition volumétrique est disposé à établir un modèle à grille en fils (22) en tant que modèle de volume, en utilisant les positions desdites images en coupe transversale (7) et desdites lignes de contour (14) au-dedans desdites images en coupe transversale (7).

12. Appareil selon la revendication 11,
**caractérisé en ce**
**que** ledit moyen de mouvement pour le mouvement dudit moyen d'exploration d'images (4) comprend un moteur pas à pas et une logique de commande, qui commandent l'acquisition pas à pas des plans d'acquisition (9) de l'objet (1).

13. Appareil selon la revendication 11 ou 12,
**caractérisé en ce que** ledit moyen d'exploration d'images (4) comprend un détecteur de position (23) à détecter les positions desdits plans d'acquisition (9).

14. Appareil selon une quelconque des revendications 11 à 13,
**caractérisé en ce**
**que** l'appareil comprend au plus un moyen d'extraction électrocardiographique et un moyen de déclenchement à déclencher la respiration pour une exploration dynamique des objets cardiaques (7).
